# EUROPEAN PATENT SPECIFICATION

(11) **EP 1 037 664 B1**
(45) Date of publication and mention of the grant of the patent: **29.09.2004**
(21) Application number: 98959023.7
(22) Date of filing: 10.12.1998
(51) Int. Cl.: A61K 39/02, A61K 39/112, A61K 39/108, A61K 39/106, A61K 39/102, A61K 39/095, A61K 39/10, C12N 1/21

(54) **VACCINES CONTAINING ATTENUATED BACTERIA**
IMPFSTOFFE DIE ABGESCHWÄCHTE BAKTERIEN ENTHALTEN
VACCINS CONTENANT DES BACTERIES ATTENUEES

(30) Priority: 11.12.1997 GB 9726233
(43) Date of publication of application: 27.09.2000
(73) Proprietor: Celltech Pharma Europe Limited, Slough, Berkshire SL1 3WE (GB)
(72) Inventor: CHATFIELD, Steven N., Wokingham, Berkshire RG41 5TU (GB); SYDENHAM, Mark;, Medeva Vaccine Research Unit, London SW7 2AY (GB); DOUGAN, Gordon, London SW7 2AY (GB)
(74) Representative: Campbell, Patrick John Henry
(86) International application number: PCT/GB1998/003680
(87) International publication number: WO 1999/029342

(56) References cited:
- EP-A- 0 400 958
- WO-A-94/03615
- LAZAR S W ET AL: "SurA assists the folding of Escherichia coli outer membrane proteins." JOURNAL OF BACTERIOLOGY, (1996 MAR) 178 (6) 1770-3. JOURNAL CODE: HH3. ISSN: 0021-9193., XP002099516 United States
- SCHODEL F ET AL: "Salmonellae as oral vaccine carriers." DEVELOPMENTS IN BIOLOGICAL STANDARDIZATION, (1995) 84 245-53. REF: 48 JOURNAL CODE: E7V. ISSN: 0301-5149., XP002099517 Switzerland
- KLEEREBEZEM M ET AL: "Characterization of an Escherichia coli rotA mutant, affected in periplasmic peptidyl-prolyl cis/trans isomerase." MOLECULAR MICROBIOLOGY, (1995 OCT) 18 (2) 313-20. JOURNAL CODE: MOM. ISSN: 0950-382X., XP002099518 ENGLAND: United Kingdom
- LIU S L ET AL: "Rearrangements in the genome of the bacterium Salmonella typhi." PROCEEDINGS OF THE NATIONAL ACADEMY OF SCIENCES OF THE UNITED STATES OF AMERICA, (1995 FEB 14) 92 (4) 1018-22. JOURNAL CODE: PV3. ISSN: 0027-8424., XP002099519 United States
- RUDD K E ET AL: "A new family of peptidyl - prolyl isomerases." TRENDS IN BIOCHEMICAL SCIENCES, (1995 JAN) 20 (1) 12-4. JOURNAL CODE: WEF. ISSN: 0167-7640., XP002099520 ENGLAND: United Kingdom
- LAZAR S W ET AL: "Role of the Escherichia coli SurA protein in stationary-phase survival." JOURNAL OF BACTERIOLOGY, (1998 NOV) 180 (21) 5704-11. JOURNAL CODE: HH3. ISSN: 0021-9193., XP002099521 United States

## Description

The invention relates to vaccines containing attenuated bacteria.

### Background to the invention

The principle behind vaccination is to induce an immune response in the host thus providing protection against subsequent challenge with a pathogen. This may be achieved by inoculation with a live attenuated strain of the pathogen (i.e. a strain having reduced virulence such that it does not cause the disease caused by the virulent pathogen).

Classically, live attenuated vaccine strains of bacteria and viruses have been selected using one of two different methodologies. Mutants have been created either by treatment of the organism using mutagenic chemical compounds or by repeated passage of the organism *in vitro*. However, use of either method gives rise to attenuated strains in which the mode of attenuation is unclear. These strains are particularly difficult to characterize in terms of possible reversion to the wild type strain as attenuation may reflect single (easily reversible) or multiple mutation events.

Using modem genetic techniques, it is now possible to construct genetically defined attenuated bacterial strains in which stable attenuating deletions can be created. A number of site directed mutants of Salmonella have been created using this type of technology (2, 5, 6, 12, 22, 35, 36, 37). Amongst the most comprehensively studied attenuating lesions are those in which mutations in the biosynthetic pathways have been created, rendering the bacteria auxotrophic (e.g. *aro* genes). Mutations in these genes were described as early as 1950 (1) as responsible for rendering Salmonella less virulent for mice. Several different auxotrophic mutations such as *galE*, *aroA* or *purA* have also been described previously (6, 12). Salmonella *aroA* mutants have now been well characterised and have been shown to be excellent live vaccines against salmonellosis in several animal species. In addition, in order to reduce the chances of a reversion to virulence by a recombination event mutations have now been introduced into two independent genes such as *aroA*/*purA* and *aroA*/*aroC*. Identical mutations in host adapted strains of Salmonella such as *S*. *typhi* (man) and *S*.*dublin* (cattle) has also resulted in the creation of a number of single dose vaccines which have proved successful in clinical (11, 17) and field trials (15).

In animal studies, attenuated *S*. *typhimurium* has been used as a vehicle for the delivery of heterologous antigens to the immune system (3, 8, 32). This raises the potential of the development of multivalent vaccines for use in man (9).

### Summary of the invention

The original aim of the work that led to the invention was the identification of novel genes that are involved in the virulence pathways of pathogenic bacteria, the identification and deletion of which may render the bacteria avirulent and suitable for use as vaccines. To identify attenuating lesions, random mutations were introduced into the chromosome of *S*. *typhimurium* using the transposon Tn*pho*A (18). This transposon is unique in that it is engineered to identify proteins that are expressed in or at the bacterial outer membrane; such proteins may be those involved in interaction with and uptake by host tissues. By using the natural oral route of infection to screen these mutants, those with important, *in vivo* induced, attenuating lesions in genes were identified.

One such gene identified through this work is *surA*. The *surA* gene product is known to promote the folding of extracytoplasmic proteins. Accordingly, the invention provides a vaccine comprising a pharmaceutically acceptable carrier or diluent and a bacterium attenuated by a non-reverting mutation in a gene encoding a protein which promotes the folding of extracytoplasmic proteins. The vaccine has the ability to confer protection against a homologous wild type oral challenge with the virulent bacterium. In addition, the bacterium used in the vaccine can act as a carrier for heterologous antigens such as fragment C of tetanus toxin.

### Detailed description of the invention

### Proteins that promote the folding of extracytoplasmic proteins

Periplasmic and outer membrane proteins are secreted across the cytoplasmic (inner) membrane in a mostly unfolded state, and they then fold after secretion. The folding often has enzymatic assistance to catalyse the formation of bonds necessary for the protein to reach its folded state. For example, the folding often requires the participation of enzymes that catalyse the formation of disulphide bonds or enzymes that catalyse the isomerisation of prolyl bonds (peptidyl-prolyl cis-trans isomerases or PPiases).

One known PPiase is SurA. The inventors have now shown that mutation of the *surA* gene causes attenuation of virulent bacteria and that the attenuated bacteria are useful as vaccines.

SurA was first described as being essential for the survival of *E*.*coli* in the stationary phase (33). It is a periplasmic protein. More recently, SurA has been described as belonging to a third, new family of PPiases (30), the parvulin family. Further studies have shown SurA to be involved in the correct folding of outer membrane proteins such as OmpA, OmpF, and LamB (16,24,29).

PPiases are divided into three families, the cyclophilins, FK506-binding proteins (FKBPs) and parvulins. Members of all three families have been found in *E*.*coli*. Apart from SurA, the parvulin family includes several proteins such as NifM, PrsA and PrtM.

### Bacteria useful in the invention

The bacteria that are used to make the vaccines of the invention are generally those that infect via the oral route. The bacteria may be those that invade and grow within eukaryotic cells and/or colonise mucosal surfaces. The bacteria are generally Gram-negative.

The bacteria may be from the genera Salmonella, Escherichia, Vibrio, Haemophilus, Neisseria, Yersinia, Bordetella or Brucella. Examples of such bacteria are *Salmonella typhimurium* - the cause of salmonellosis in several animal species; *Salmonella typhi* - the cause of human typhoid; *Salmonella enteritidis* - a cause of food poisoning in humans; *Salmonella choleraesuis -* a cause of salmonellosis in pigs; *Salmonella dublin* - a cause of both a systemic and diarrhoel disease in cattle, especially of new-born calves; *Escherichia coli* - a cause of diarrhoea and food poisoning in humans; *Haemophilus influenzae* - a cause of meningitis; *Neisseria gonorrhoeae* - a cause of gonnorrhoeae; *Yersinia enterocolitica -* the cause of a spectrum of diseases in humans ranging from gastroenteritis to fatal septicemic disease; *Bordetella pertussis* - the cause of whooping cough; or *Brucella abortus -* a cause of abortion and infertility in cattle and a condition known as undulant fever in humans.

Salmonella bacteria are particularly useful in the invention. As well as being vaccines in their own right against infection by Salmonella, attenuated Salmonella can be used as carriers of heterologous antigens from other organisms to the immune system via the oral route. Salmonella are potent immunogens and are able to stimulate systemic and local cellular and antibody responses. Systems for driving expression of heterologous antigens in Salmonella *in vivo* are known; for example the *nirB* and *htrA* promoters are known to be effective drivers of antigen expression *in vivo.*

The invention is also particularly applicable to *E.coli,* especially exterotoxigenic *E*. *coli* ("ETEC"). ETEC is a class of *E*.*coli* that cause diarrhoea. They colonise the proximal small intestine. A standard ETEC strain is ATCC H10407.

Infections of ETEC are the single most frequent cause of travellers diarrhoea, causing 3-9 million cases per year amongst visitors to developing countries. In endemic areas, ETEC infections are an important cause of dehydrating diarrhoea in infants and young children, resulting in 800,000 deaths a year in the under fives wold-wide. In developing countries, the incidence of ETEC infections leading to clinical disease decreases with age, indicating that immunity to ETEC infection can be acquired. In contrast, naive adults from industrialized countries who visit endemic areas are highly susceptible to ETEC infections. However, with prolonged or repeated visits to endemic areas susceptibility to ETEC infections diminishes, suggesting that a live attenuated approach to ETEC vaccination may prove successful.

Seq. Id. No. 1 shows the sequence of the *surA* open reading frame in *Salmonella typhimurium*, and Seq. Id. No. 2 shows the sequence of the *surA* open reading frame in *E*. *coli.*

### Second mutations

The bacteria used in vaccines of the invention preferably contain a mutation in one or more genes in addition to the mutation in the gene encoding a protein which promotes folding of extracytoplasmic proteins. This is so that the risk of the bacterium reverting to the virulent state is minimised, which is clearly important for the use of the bacterium as a human or animal vaccine. Although bacteria containing only a mutation in a protein which promotes folding of extracytoplasmic proteins are attenuated and the risk of reversion is small, it will generally be desirable to introduce at least one further mutation so as to reduce the risk of attenuation yet further.

A number of genes that are candidates for second and further mutations are known (see e.g. ref 39). These include the *aro* genes (35), the *pur* genes, the *htrA* gene (37), the *ompR* gene (36), the *galE* gene, the *cya* gene, the *crp* gene or the *phoP* gene. The *aro* gene may be *aroA*, *aroC*, *aroD* or *aroE*. The *pur* gene may be *purA*, *purB*, *purE or purH*. The use of *aro* mutants, especially double *aro* mutants, is preferred because such mutants have been shown to be particularly effective as vaccines. Suitable combinations of *aro* mutations are *aroAaroC*, *aroAaroD* and *aroAaroE*.

### The nature of the mutation

The mutations introduced into the bacterial vaccine generally knock-out the function of the gene completely. This may be achieved either by abolishing synthesis of any polypeptide at all from the gene or by making a mutation that results in synthesis on non-functional polypeptide. In order to abolish synthesis of any polypeptide, either the entire gene or its 5'-end may be deleted. A deletion or insertion within the coding sequence of a gene may be used to create a gene that synthesises only non-functional polypeptide (e.g. polypeptide that contains only the N-terminal sequence of the wild-type protein). In the case of mutations in genes encoding proteins which promote the folding of extracytoplamic proteins, the mutation generally abolishes the ability of the protein to promote such protein folding.

The mutations are non-reverting mutations. These are mutations that show essentially no reversion back to the wild-type when the bacterium is used as a vaccine. Such mutations include insertions and deletions. Insertions and deletions are preferably large, typically at least 10 nucleotides in length, for example from 10 to 600 nucleotides.

The bacterium used in the vaccine preferably contains only defined mutations, i.e. mutations which are characterised. It is clearly undesirable to use a bacterium which has uncharacterised mutations in its genome as a vaccine because there would be a risk that the uncharacterised mutations may confer properties on the bacterium that cause undesirable side-effects.

The attenuating mutations may be constructed by methods well known to those skilled in the art (see ref 31). One means for introducing non-reverting mutations into extracytoplamic proteins is to use transposon Tn*phoA*. This can be introduced into bacteria to generate enzymatically active protein fusions of alkaline phosphatase to extracytoplasmic proteins. The Tn*phoA* transposon carries a gene encoding kanamycin resistance. Transductants are selected that are kanamycin resistant by growing colonies on an appropriate selection medium.

Alternative methods include cloning the DNA sequence of the wild-type gene into a vector, e.g. a plasmid or cosmid, and inserting a selectable marker into the cloned DNA sequence or deleting a part of the DNA sequence, resulting in its inactivation. A deletion may be introduced by, for example, cutting the DNA sequence using restriction enzymes that cut at two points in the coding sequence and ligating together the two ends in the remaining sequence. A plasmid carrying the inactivated DNA sequence can be transformed into the bacterium by known techniques. It is then possible by suitable selection to identify a mutant wherein the inactivated DNA sequence has recombined into the chromosome of the bacterium and the wild-type DNA sequence has been rendered non-functional in a process known as homologous recombination.

### Expression of heterologous antigens

The attenuated bacterium used in the vaccine of the invention may be genetically engineered to express an antigen from another organism (a "heterologous antigen"), so that the attenuated bacterium acts as a carrier of the antigen from the other organism. In this way it is possible to create a vaccine which provides protection against the other organism. A multivalent vaccine may be produced which not only provides immunity against the virulent parent of the attenuated bacterium but also provides immunity against the other organism. Furthermore, the attenuated bacterium may be engineered to express more than one heterologous antigen, in which case the heterologous antigens may be from the same or different organisms.

The heterologous antigen may be a complete protein or a part of a protein containing an epitope. The antigen may be from another bacterium, a virus, a yeast or a fungus. More especially, the antigenic sequence may be from tetanus, hepatitis A, B or C virus, human rhinovirus such as type 2 or type 14, herpes simplex virus, poliovirus type 2 or 3, foot-and-mouth disease virus, influenza virus, coxsackie virus or *Chlamydia trachomatis*. Useful antigens include *E*.*coli* heat labile toxin B subunit (LT-B), *E*.*coli* K88 antigens, P.69 protein from *B*. *pertussis* and tetanus toxin fragment C.

The DNA encoding the heterologous antigen is expressed from a promoter that is active *in vivo*. Two good promoters are the *nirB* promoter (38, 40) and the *htrA* promoter (40).

A DNA construct comprising the promoter operably linked to DNA encoding the heterologous antigen may be made and transformed into the attenuated bacterium using conventional techniques. Transformants containing the DNA construct may be selected, for example be screening for a selectable marker on the construct. Bacteria containing the construct may be grown *in vitro* before being formulated for administration to the host for vaccination purposes.

### Formulation of the vaccine

The vaccine may be formulated using known techniques for formulating attenuated bacterial vaccines. The vaccine is advantageously presented for oral administration, for example in a lyophilised encapsulated form. Such capsules may be provided with an enteric coating comprising, for example, Eudragate "S" (Trade Mark), Eudragate "L" (Trade Mark), cellulose acetate, cellulose phthalate or hydroxypropylmethyl cellulose. These capsules may be used as such, or alternatively, the lyophilised material may be reconstituted prior to administration, e.g. as a suspension. Reconstitution is advantageously effected in a buffer at a suitable pH to ensure the viability of the bacteria. In order to protect the attenuated bacteria and the vaccine from gastric acidity, a sodium bicarbonate preparation is advantageously administered before each administration of the vaccine. Alternatively, the vaccine may be prepared for parenteral administration, intranasal administration or intramuscular administration.

The vaccine may be used in the vaccination of a host, particularly a human host but also an animal host. An infection caused by a microorganism, especially a pathogen, may therefore be prevented by administering an effective dose of a vaccine prepared according to the invention. The dosage employed will be dependent on various factors including the size and weight of the host and the type of vaccine formulated. However, a dosage comprising the oral administration of from 10⁷ to 10¹¹ bacteria per dose may be convenient for a 70kg adult human host.

### Examples

The following Examples serve to illustrate the invention.

### Brief description of the drawings

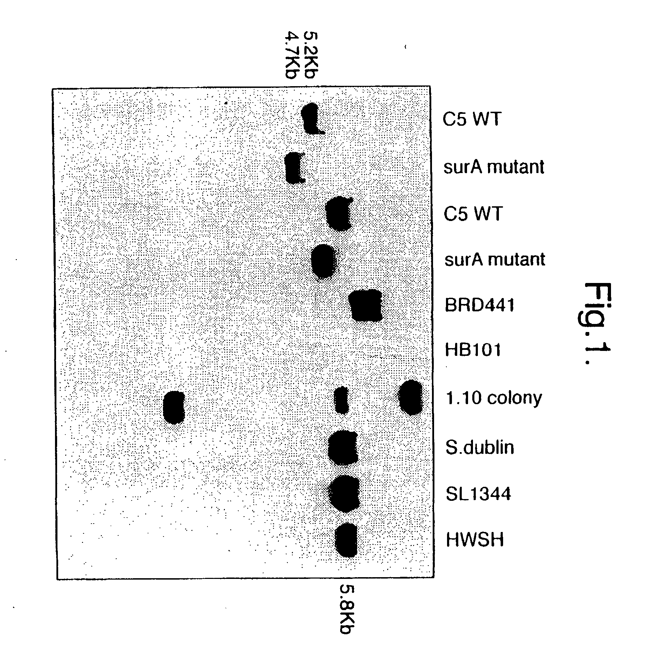

Figure 1: Southern blot confirming the defined deletion created within *surA* in the strain BRD 1115. Lanes 1 and 2 have been restricted using the enzyme *Pst*I, lanes 3-10 have been restricted with *Sal*I. The filters have been probed using a 500 bp PCR product that contains a 500 bp fragment from the middle of the *surA* gene. Lanes 2 and 4 show hybridisation of this probe to a band 500 bp smaller than the corresponding wild type lanes 1 and 3. The transposon mutant BRD441 shows hybridisation to 2 bands since the enzyme *Sal*I cuts the transposon into two. HB101 shows no hybridisation whilst the other wild type Salmonella strains show the same hybridisation as C5 when restricted with *Sal*I.

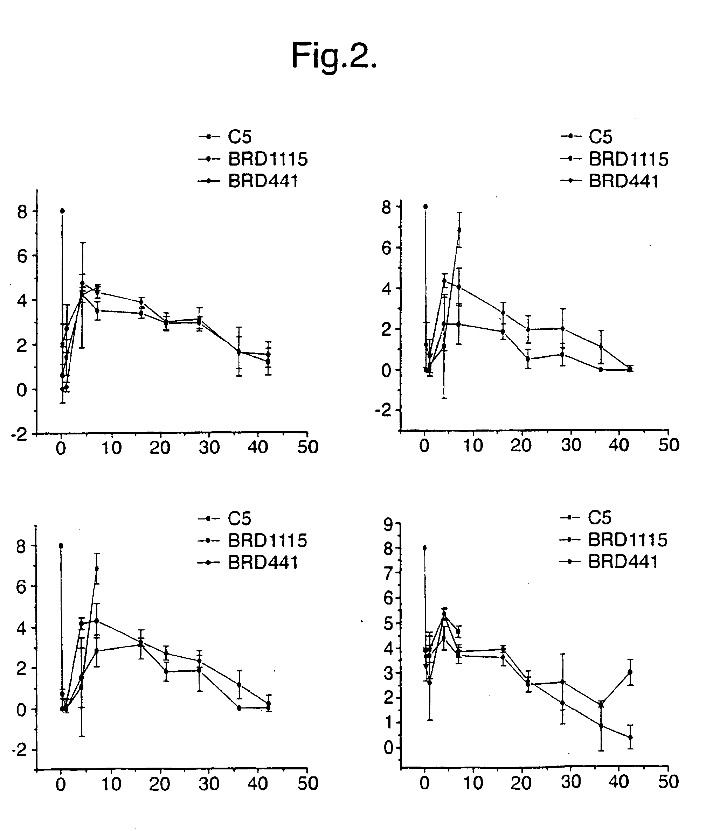

Figure 2: This figure shows the colonisation and persistence of BRD1115, BRD441 and the wild type C5 in the mesenteric lymph nodes (top left graph), Peyer's patches (bottom right), spleens (bottom left) and livers (top right) in BALB/c mice following oral inoculation. The x-axis is time in days and the y-axis is log₁₀ CFU/ml (CFU stands for colony forming units).

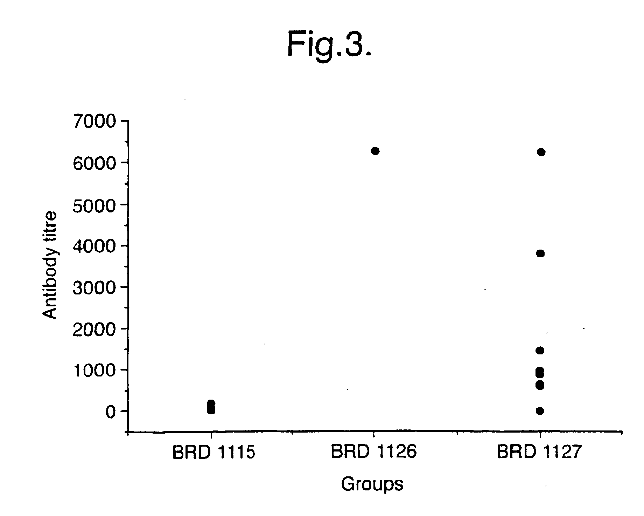

Figure 3: Three strains were constructed to evaluate the ability of mutant Salmonella strains to deliver the heterologous antigen Fragment C in the mouse. BRD1115 is the parental strain. Two plasmids encoding the Fragment C gene of tetanus toxin under the control of either the *htrA* or *nirB* promoter were introduced into the strain BRD1115 to give the strains BRD1127 and 1126 respectively. Expression of fragment C was determined *in vitro* by Western blotting. These strains were then used in an *in vivo* experiment using BALB/c mice. Groups of 10 mice were immunised orally with log₁₀8 organisms each of the 3 strains. Serum samples were taken weekly and analysed for total antibodies against tetanus toxin fragment C. The titres of anti-fragment C were determined as the highest sample dilution giving an absorbance value of 0.3 above normal mouse serum. The highest sample dilution tested was 1/6250. All mice immunised with BRD 1126 showed antibody titres higher than 6250.

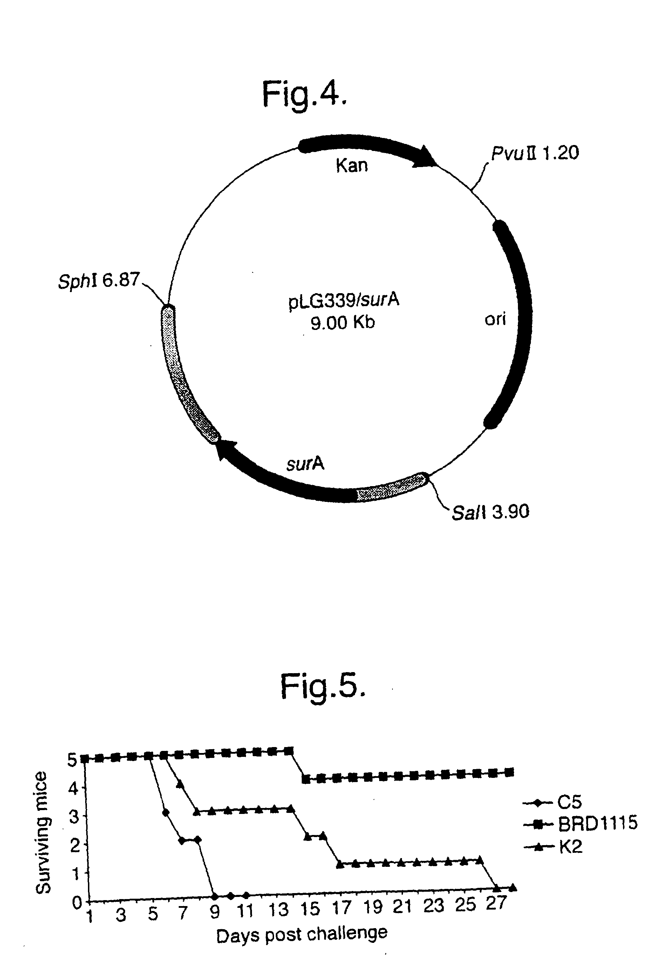

Figure 4: Schematic showing a plasmid map of pLG339/*surA*.
Figure 5: Graph showing the survival of Balb/c mice following oral challenge with log₁₀8 bacteria of the three strains C5, BRD 1115 and K2.

### Example 1

This Example shows the identification of mutations in *surA* as attenuating mutations, the construction of a defined *surA* mutation and the evaluation of a *surA* mutant as a vaccine (both against homologous challege and as a carrier for heterologous antigens).

### Materials and methods

### 1.1 Bacteria, bacteriophage, plasmids and growth conditions

The bacteria used in this study are listed in Table 1. Bacteria were routinely cultured on L-agar or in L-broth containing 100µg/ml ampicillin or 50µg/ml kanamycin where appropriate. The bacteriophage P22HT105/1int⁻ is a high frequency transducing bacteriophage obtained from Dr Tim Foster (Trinity College, Dublin). The plasmid pGEM-T (Promega Corporation, USA) is designed for direct cloning of PCR fragments and pBluescript^{ò}II SK+ (Stratagene Ltd, Cambridge, U.K.) is a general cloning vector. The other plasmids are described in the text.

### 1.2 Purification of DNA and DNA manipulation techniques

All DNA manipulation including Southern blotting were carried out as described by Sambrook *et al* (31). Restriction enzymes and T4 DNA ligase were purchased from Boehringer Mannheim (Lewes, UK) and used according to the manufacturers instructions. Chromosomal DNA preparation was prepared according to the method of Hull (13).

### 1.3 DNA sequencing

Double stranded plasmid sequencing was carried out using the Sequenase kit (Trade Mark, United States Biochemical Corporation) according to the manufacturers' instructions. Labelling of the DNA was achieved using ³⁵S-dATP (Amersham, UK) and fragments separated on an 8% acrylamide/bis-acrylamide gel containing 7M urea, for 2hours at 35 mA.

### 1.4 DNA amplification by polymerase chain reaction

Polymerase chain reactions (PCR) were carried out with *Taq* DNA polymerase using the GeneAmp kit (Trade Mark, Perkin Elmer Cetus, USA) according to the manufacturers' instructions. Oligonucleotides were purchased from the Molecular Medicine Unit, Kings College, London and the sequences are shown in Table 1. Mixtures of DNA and specific primers were subjected to multiple rounds of denaturation, annealing and extension in the presence of the enzyme *Taq* polymerase. 100 ng plasmid DNA and lmg chromosomal DNA were added to a mixture containing 5µl 10 x buffer (100mM Tris-HCl, pH 8.3: 500mM KCl; 15mM Mg Cl₂; 0.01% gelatine(v/v)); 8µl of deoxy-nucleotide mixture (1.25mM each of deoxy-nucleotide triphosphate; dATP, dCTP, dGTP and dTTP); 1µl of a 10µM sense primer; 1µl of a 10µM anti-sense primer and 2.5 units *Taq* polymerase. This mixture is overlaid with 50µl light mineral oil (Sigma) to prevent evaporation and the tubes incubated in an Omnigene Thermal Cycler (Trade Mark, Hybaid). Amplification of the DNA was performed using the following programme: 1 cycle of 95°C for 5 minutes, 50°C for 1.5 minutes, 74°C for 2 minutes; 19 cycles of 95°C for 1.5 minutes, 50°C for 2 minutes, 74°C for 3 minutes; 10 cycles of 95°C 2 minutes, 50°C for 2 minutes, 74°C for 7 minutes.

### 1.5 Transformation of bacteria

### 1.5.1. Heat shock

Bacteria are rendered competent to DNA uptake by the calcium chloride method. An overnight bacterial culture was used to seed a fresh 25 ml LB broth culture (a 1:100 dilution) which was grown aerobically with shaking until the cells reached mid-log growth phase (OD 650nm = 0.4 to 0.6). The cells were harvested by centrifugation at 3000 x g for 10 minutes at 4°C. The supernatant was discarded and the pellet resuspended in 25 ml ice-cold 75mM CaCl₂. The process was repeated and the cells incubated on ice for 30 minutes. The cells were pelleted by centrifugation at 3000 x g for 10 minutes at 4°C. The cell pellet was resuspended in 1.2 ml ice-cold 75mM CaCl₂ and stored on ice until needed. The cells were then competent to DNA uptake. A maximum of 20µl of the ligation mix was added to 200µl of the competent cells and the mixture stored on ice for 30 minutes. The cells were then subjected to heat shock by incubation in a 42°C waterbath for 2 minutes. The cells were then transferred back to ice for a further 2 minutes. 1 ml of LB broth was added to the mixture and the cells incubated at 37°C for at least 60 minutes to allow expression of the antibiotic marker on the plasmid. 100µl aliquots of cells were plated onto LB agar plates containing the appropriate antibiotics and incubated overnight at 37°C.

### 1.5.2. Electroporation

Plasmid DNA was introduced into bacterial strains using electroporation. Mid-log phase growth cultures were generated as for the heat-shock method and the cells pelleted by centrifugation at 3000 x g for 10 minutes at 4°C. The cell pellet was washed twice with an equal volume of ice-cold 10% glycerol and pelleted as before. The cell pellet was resuspended in 300-500µl ice-cold 10% glycerol. Approximately 100 ng plasmid (or 1 µg suicide vector) in a volume not greater than 6 µl sterile water was added to 60 µl competent cells in a pre-chilled electroporation cuvette on ice. The plasmid was electroporated into the bacteria using a Bio-Rad Gene Pulser (Trade Mark) with the following conditions 1.75kV, 600Ω, 25µF. 1ml LB broth was then added to the contents of the electroporation cuvette and the mixture incubated at 37°C for 90 minutes to allow the cells to recover. 100 µl aliquots of the electroporation mix were plated out onto selection media and incubated at 37°C overnight.

### 1.6 P22 Transduction

Transduction experiments were carried out using the bacteriophage P22 HT105/1 int⁻. Phage lysates were prepared using LB5010 as the donor strain. A 5ml overnight culture of LB5010 was grown in L broth containing 0.2% glucose and galactose to increase the expression of phage receptors on the cell surface. Ten fold serial dilutions of the P22 stock were made in TMGS up to 10⁻⁸ (stock is approximately 10¹⁰ pfu/ml). 10µl of each dilution was added to 100µl of the overnight stock of cells and incubated at 37°C for 30-45 minutes to allow adsorbtion of the phage to the cells. 3mls of top agar was added to each incubation and spread onto L agar plates containing 100µg/ml ampicillin. The plates were incubated at 37°C for approximately 4-5 hours until plaques were visible. The dilution that gave almost confluent plaques after this length of time was the one chosen for harvesting. The plaques were harvested by scraping the top agar into 2ml of phage buffer with a glass microscope slide. A few drops of chloroform were added and the phage stock stored at 4°C until needed. The recipient strain C5 was grown during the day in L broth at 37°C until late log/stationary phase. 1µl, 5µl, 10µl, 20µl, and 50µl aliquots of the new phage stock were added to 100µl aliquots of the recipient strain and incubated at 37°C for 1 hour. The cells were then spread onto L agar ampicillin plates containing 5mM EGTA (to prevent phage replication) and incubated at 37°C overnight. Colonies were replated onto L agar ampicillin plates containing 5mM EGTA three times to ensure that they were free from phage. The colonies no longer had a jagged appearance thus indicating an absence of phage.

### 1.7 In vitro analysis of bacterial strain

### 1.7.1. Agglutination with antisera

Agglutination using anti-sera raised against the O antigen of *Salmonella* can be used as a rapid test not only for the integrity of the bacterial LPS but also as a diagnostic of the strain, e.g. anti-sera against the 04 and 05 antigens for *S*.*typhimurium*. These were obtained from Murex Diagnostics Ltd (Dartford U.K.). A sweep of colonies was harvested from the growth on a plate incubated overnight, and resuspended in 100µl PBS. This sample was mixed with a drop of antisera on a glass slide and the agglutination compared with a positive and negative sample.

### 1.7.2 HEp-2 invasion assay

The HEp-2 cell line is an adherent epidermoid carcinoma derived from human larynx (ATCC CCL23). It can be cultured as a monolayer in Dulbecco's modified Eagle's medium with 10% FCS, glutamine and penicillin/streptomycin at 37°C in the presence of 5% CO₂. Confluent cells were detached from the tissue culture flasks by the use of trypsin/EDTA. The cells were first washed in PBS to remove any serum that might affect the action of the trypsin. Trypsin/EDTA was then added to the monolayer and the cells incubated at 37°C for 5 minutes. The cells were removed from the plastic by gentle tapping on the edge of the flask. The trypsin was neutralised with 1.5 volumes of DMEM. Cells are collected by centrifugation at 1000 x g for 5 minutes. The supernatant was removed and the cell pellet resuspended in DMEM. The cell pellet was counted and the concentration adjusted to give 2x 10⁵cells per ml.

1 ml of the cell suspension was added to one well of a 24 well tissue culture plate (Costar 3524), three wells for each bacterial strain being investigated. The cells were incubated overnight to form a confluent monolayer in the well. The cells were then washed 5 times with PBS to ensure removal of the antibiotics and 1 ml DMEM added (without any antibiotics). 1x10⁷ bacteria were added to each well and incubated at 37°C for 3 hours. The cells were washed 3 times with PBS to remove any extracellular bacteria. 1ml of DMEM containing 100µg/m gentamycin was added and the cells incubated for a further 1 hour. The cells were washed 5 times with PBS. The cells were lysed by the addition of 1ml of 0.1% Triton-X-100 at 37°C for 15 minutes. The cells were further lysed by agitation with a blue pipette tip and the lysate transferred to a 1.5ml centrifuge tube. The viable bacteria that had invaded the cells were counted using the Miles-Misra drop test method (19).

### 1.8. In vivo analysis of bacterial strains

### 1.8.1. Preparation of live bacteria for immunisation of mice.

A vial of the appropriate strain was thawed from liquid nitrogen and used to inoculate a 250 ml culture of LB broth containing antibiotic where appropriate. The culture was grown overnight at 37°C without shaking. The bacteria were harvested by centrifugation at 3000 x g for 10 minutes and washed once in sterile PBS. The bacteria were harvested again by centrifugation and resuspended in 5 ml sterile PBS. The concentration of bacteria was estimated by optical density at 650 nm using a standard growth curve for that strain. Based on this estimate the cell concentration was adjusted with PBS to that required for immunisation. A viable count was prepared of each inoculum to give an accurate number of colony forming units per ml (cfu/ml) administered to each animal.

### 1.8.2. Oral immunisation of mice with live bacteria.

The mice were lightly anaesthetised with a mixture of halothane and oxygen and the bacteria administered by gavage in 0.2 ml volumes using a gavage needle attached to a 1ml syringe.

### 1.8.3. Intravenous (i.v.) immunisation of mice with live bacteria.

Mice were placed in a warm chamber and 0.2 ml volumes injected into a tail vein of each mouse using a 27 gauge needle.

### 1.8.4. Enumeration of viable bacteria in mouse organs.

Groups of four or five mice were sacrificed up to 7 weeks post oral immunisation with three bacterial strains. Spleens, livers, mesenteric lymph nodes and Peyer's patches were removed and homogenised in 10ml sterile PBS using a stomacher (Colworth, U.K.). Dilutions of these homogenates were plated out in LB agar with kanamycin if required and incubated overnight at 37°C. The number of viable bacteria present in each homogenate was then calculated from the dilution.

### 1.9. Determination of antibody titres against fragment C.

Serum antibody responses against fragment C were measured by enzyme-linked immunosorbant assay (ELISA) as previously described (28) using 96 well EIA/RIA plates (Costar 3590). Absorbance values were read at A₄₉₀ and plotted against dilutions (data not shown). A normal mouse serum control was added to each ELISA plate and used to define the background level response.

### 1.10 Tetanus toxin challenge

Mice were challenged with 0.05 µg (50 x 50% lethal doses) of purified tetanus toxin as previously described (7), and fatalities recorded for 4 days.

### Results

### 2.1 Cloning and mapping of TnphoA insertion sites

A number of *S*.*typhimurium* Tn*phoA* insertion mutants were previously identified as being attenuated when administered orally to BALB/c mice. In addition some of these mutants also exhibited a reduced ability to invade the cultured epithelial cell line HEp-2. To identify the genes that had been disrupted by the Tn*pho*A insertion, genomic DNA was digested using *Sau* 3A and cosmid banks prepared from each strain. These banks were screened using Tn*phoA* probes and cosmids exhibiting homology with the 3' and 5' probes were examined. Fragments from these cosmids were cloned into the vector pBluescript^{ò}II SK+. The nucleotide sequence surrounding these insertion sites was determined and the genes identified. Two insertions were found to be within the *htrA* gene (14), one in the *osmZ* gene (10) and one in the *surA* gene.

The *surA* gene open reading frame of *Salmonella typhimurium* shown in Seq Id No. 1 is 1281 bases long, encoding a protein of some 427 amino acids with a molecular weight of 47.2Kd. This protein is virtually identical to that found in *E*.*coli* (34), and is described as being essential for survival in long term culture (33). The *surA* gene contains a leader peptidase cleavage site indicating that this is a transported protein. It has now been described as belonging to a peptidyl prolyl isomerase family, with a function to aid the correct folding of outer membrane proteins (16, 24, 29).

### 2.2 Introduction of a defined deletion into the surA gene.

Restriction analysis and DNA sequencing of the *surA* gene revealed the presence of single *Hpa*I and *Sma*I restriction enzyme sites within the coding region of the gene which could be used to generate a deletion of 400 bases. The plasmid pGEM-T/212/213 was constructed containing a 3Kb region encompassing the entire *surA* gene and flanking region. Digestion of the plasmid with the enzymes *Hpa*I and *Sma*I, gel purification of the large 5.5Kb fragment and re-ligation resulted in a plasmid containing a 419bp deletion within the *surA* gene. This plasmid was designated pGEM-T/ΔsurA.

### 2.3 Introduction of the surA deletion into the chromosome of S.typhimurium C5.

The plasmid pGEM-T was digested with the two restriction enzymes *Sph* I and *Sal*I. The 2.6kb fragment containing the deleted *surA* gene was gel purified and ligated into the suicide replicon pGP704 that had previously been digested with the same enzymes. The suicide replicon pGP704 has been used previously to introduce deletions into the chromosome of *S*.*typhi* (4) and *S*.*typhimurium* (26) which lack the *pir* gene, the product of which is essential for the replication of pGP704. The ligation mix was used to transform the strain SY327, an *E*.*coli* strain that contains the *pir* gene, and a plasmid of the expected size identified by restriction analysis. This plasmid was designated pGP704/ΔsurA. Since suicide replicons cannot replicate in *S*.*typhimurium* the drug resistance marker is only expressed if there has been a single homologous recombination event, incorporating the plasmid into the bacterial chromosome.

The plasmid pGP704/ΔsurA was used to transform the semi-rough *S*.*typhimurium* strain LB5010 by the calcium chloride method. Three transformants were selected on agar containing ampicillin. These single crossovers were moved from this intermediate strain into the wild type C5 using P22 transduction (20). P22 lysates were prepared from the three transductants and introduced into C5. One ampicillin resistant colony was obtained from this process. This transformant was sub-cultured twice into L-broth containing no selection and grown for 48 hours. Serial dilutions of this culture were made and the 10⁻⁶ dilution was spread onto L-agar plates containing no selection. 500 colonies were streaked by hand on to duplicate plates, one containing agar, the other agar with ampicillin. One colony was found to be ampicillin sensitive indicating the loss of the drug resistance marker of the plasmid following a second homologous recombination event.

This potential *surA* mutant was confirmed as a *S*.*typhimurium* strain by agglutination with 04 and 05 antiserum. The deletion was confirmed by PCR using the primers MGR92 and MGR93, giving a 1 kb product. The deletion was also confirmed cloning the PCR product into the vector pGEM-T to give the plasmid pGEM-T/92/93, and sequencing across the deletion using the primers MGR130 and 135. Figure 1 shows the results of probing *Pst*I and *Sal*I digested genomic DNA from C5 and the *surA* mutant strain with a PCR product obtained from the wild type C5. The band seen in the *surA* mutant track is approximately 400 bases smaller than that seen in the wild type. This deleted strain was designated BRD1115.

### 2.4 Characterisation of the strain BRD1115

### 2.4.1 In vitro analysis of the invasion of cultured epithelial cells

The strain BRD1115 was tested for its ability to invade the cultured epithelial cell line HEp-2. The levels of invasion were found to be reduced by 80% in comparison to the wild type strain C5. The transposon mutant BRD441 showed a 90% reduction in invasion compared to C5.

### 2.4.2. Evaluation of the in vivo properties of BRD1115 in BALB/c mice.

### 2.4.2.1. Determination of oral and i.v. LD50's

The oral and i.v. LD₅₀'s of BRD 1115, C5 and BRD441 were calculated using the mouse susceptible strain BALB/c. 5 mice per group were inoculated either orally or i.v. with doses ranging from log₁₀4 to log₁₀ 10 orally and log₁₀1 to log₁₀5 i.v. Deaths were recorded over 28 days and the LD₅₀'s calculated by the method of Reed and Meunch (27). BRD1115 was determined to show nearly 5 logs of attenuation orally and 3.5 logs i.v compared to C5. BRD441 showed 4.5 logs attenuation orally and 1 log i.v.. The results are presented in Table 2.

### 2.4.2.2. Persistence of strains in the organs of BALB/c mice following oral inoculation

Groups of 4 BALB/c mice were orally inoculated with log₁₀8 organisms of the three strains. Mice were killed at days 0,1,4,7,10,16,21 and 28 and the organs examined for bacterial load. The wild type strain C5 colonised the spleen, liver, mesenteric lymph nodes and Peyer's patches in high numbers (>log₁₀4 cfu/ml), eventually resulting in the death of the animals. BRD 1115 and BRD 441 on the other hand persisted in the liver and spleens for more than 40 days in low numbers (<log₁₀2 cfu/ml). These results are presented in Figure 2.

### 2.5. Evaluation of BRD1115 as a potential vaccine strain

### 2.5.1. BRD 1115 protects against homologous challenge

Groups of BALB/c mice were orally immunised with log₁₀8 organisms of BRD1115 and challenged with the wild type strain C5 at 4 weeks and 10 weeks post inoculation. The mice were challenged with log₁₀4 to log₁₀10 organisms C5 and a new oral LD₅₀ calculated. The levels of protection are presented in Table 3, showing log₁₀4 protection after 4 weeks and log₁₀5 after 10 weeks.

### 2.5.2. BRD1115 as a potential carrier strain for heterologous antigens

Two plasmids encoding the C fragment of tetanus toxin were introduced into two isolates of BRD1115 by electroporation. The plasmids are pTET*nir*15 (38) in which fragment C is under the control of the *nirB* promoter, and pTET*htrA* in which fragment C is under the control of the *htrA* promoter. The plasmids were found to be maintained at levels greater than 90% in BRD1115 even when the selection pressure of ampicillin was removed from the growth medium. *In vitro* expression of fragment C was determined by Western blotting. The strains were cultured under both inducing (42°C for BRD 1126 and anaerobiosis for BRD 1127) and non-inducing conditions (37°C for BRD 1126 and aerobiosis for BRD 1127). A higher level of expression was seen for both strains under inducing conditions with BRD 1127 showing higher levels of fragment C expression than BRD 1126.

Groups of 10 BALB/c mice were orally immunised with log₁₀8 organisms and bled weekly. The titres of anti-fragment C antibodies present in the serum of each animal was determined by ELISA. The titres were determined as the reciprocal of the highest sample dilution giving an absorbance of 0.3 above normal mouse serum. The results are presented in Figure 3.

Four weeks post immunisation the mice were challenged with 50LD₅₀'s of tetanus toxin subcutaneously and the deaths noted over 4 days. The results are presented in Table 4, showing that 100% protection was given after immunisation with BRD 1127 (fragment C under the control of the *htrA* promoter) and 60% protection after immunisation with BRD1126 (under *nirB* promoter). No naive mice survived the challenge.

### Example 2

This Example confirms that the mutation in *surA* is responsible for the attenuation. This was determined by complementation of the deleted gene with an intact version of the gene expressed on a plasmid. The complemented strain was as virulent as the wild-type organism given orally to mice.

### Materials and Methods

### 3.1 Construction of plasmid containing the intact surA gene

pLG339 (41) is a low copy number plasmid based on pSC105. A 3kb fragment of the plasmid pGEM-T/212/213 (section 2.2) containing the intact *surA* gene and flanking region was cloned into the *Sph*I/*Sal*I sites of the plasmid pLG339 to create the plasmid pLG339/*surA*. A schematic of this plasmid is shown in Figure 4.

### 3.2 Introduction of the plasmid pLG339/surA into defined mutant strain BRD1115

The plasmid was electroporated into electrocompetent BRD 1115 as previously described in section 1.5.2. Transformants containing the plasmid were selected by plating the electroporation mix onto agar plates containing 15µg/ml kanamycin. Plasmid DNA was recovered from a single colony of this transformation and checked for identity by restriction analysis. This strain was called K2.

### 3.3 Plasmid stability within the strain K2.

The ability of the intact *surA* gene on the plasmid to complement the action of the deleted *surA* gene in the chromosome relies on the plasmid being retained within the bacterial strain. The plasmid contains the gene encoding resistance to the antibiotic kanamycin. Culturing the strain in the presence of the antibiotic should ensure that the plasmid is retained. However it is important that the plasmid be retained in the absence of the antibiotic selection as antibiotic selection is not possible *in vivo*.

A single colony of the strain K2 was inoculated into duplicate 10 ml cultures of L broth with and without kanamycin. The cultures were grown with shaking at 37°C for a total of 72 hours. Samples were taken at 30 and 48 hours post inoculation and serial dilutions plated onto L agar plates with and without kanamycin. The cultures were diluted 1/100 into Fresh L broth with and without kanamycin and cultured for a further 24 hours. Dilutions of the culture were again plated out onto L agar plates with and without kanamycin. Numbers of colony forming units (cfu) were recorded and are reported in Table 5.

### 3.4 Oral immunisation of mice with the strain K2.

The strain K2 was grown as described in 1.8 and used to challenge orally groups of 5 Balb/c mice (as previously described) with a dose range from 10⁴ to 10¹⁰ /dose. Deaths were recorded over 28 days and the LD₅₀s calculated according to the method of Reed and Meunch (described in 2.3.2).

### Results

### 4.1 Strain

The plasmid pLG339/*surA* was recovered from the strain K2 and digested with the two enzymes *Sph*I and *Sal*I. Separation of the resultant bands by agarose gel electrophoresis revealed the correct sized bands of 6.2 and 3 kb.

### 4.2 Plasmid Stability

The presence of the plasmid pLG339/*surA* was investigated in the strain K2. The results show that in the absence of antibiotics the plasmid is retained by the bacteria. In these studies, at least 82% of the bacteria retain the plasmid when grown without antibiotics. This suggests that this plasmid should be maintained when the bacteria are used to infect mice.

### 4.3 Complementation data

Groups of 5 Balb/c mice were orally challenged with various doses of the putative complemented strain K2. The oral LD₅₀ of the complemented strain K2 was calculated to be log₁₀4.35 compared to that of log₁₀4.17 for the parental strain C5.

Deaths of the mice within the group of mice challenged with log₁₀8 bacteria of the three strains C5, BRD1115 and K2 are represented in Figure 5. Although the *surA* gene expressed from the plasmid appears to commplement the defined mutation *in vivo*, the apparent delay in the time to death (when compared to the wild type parent strain) suggests the level of *surA* expression may be reduced in the strain K2.

**Table 1:**

| Bacterial strains, plasmids and oligonucleotide primers used in this study | | |
|---|---|---|
| Bacterial strains | Properties | Source or ref |
| *E*.*coli* | | |
| SY327 | λ*pir* lysogen | Miller V.L.(23) |
| | | |

| *S*.*typhimurium* | | |
|---|---|---|
| LB5010 | semi-rough | the inventor laboratory |
| C5 | wild type | C.Hormaeche, Cambridge, U.K. |
| BRD441 | TnphoA mutant, kan^{R} | Miller I (21) |
| BRD 1115 | | this study |
| BRD 1126 | amp^{R} | Oxer M.D. (25) |
| BRD 1127 | amp^{R} | in press |
| | | |

| Plasmids | | |
|---|---|---|
| pBluescript^{ò}II SK+ | amp^{R} | Stratagene Ltd |
| pGEM-T | amp^{R} | Promega Corp. |
| pGP704 | amp^{R} | Miller V.L. (23) |
| pGEM-T/212/213 | amp^{R} | this study |
| pGEM-T/ΔsurA | amp^{R} | this study |
| pGP704/ΔsurA | amp^{R} | this study |
| pGEM-T/92/93 | amp^{R} | this study |
| pTET*nir*15 | amp^{R} | Oxer M.D. (25) |
| pTET*htr*A | amp^{R} | in press |
| | | |

| Oligo Primers | | |
|---|---|---|
| MGR 92 | TCGGCACGCAAGAAATGT | Kings College, London |
| MGR 93 | AGACGACCAGTTCAATCG | " " " |
| MGR 130 | CGATGGGCTGAACTATTC | " " " |
| MGR 135 | TATGCAGCTTCGTTAGCG | " " " |

**Table 2:**

| | | |
|---|---|---|
| The oral and i.v. LD ₅₀'s of the three strains C5, BRD 441 and BRD 1115 were determined in BALB/c mice. Groups of 5 mice were immunised with doses ranging from log₁₀4 to log₁₀10 cfu of the strains BRD 441 and BRD 1115, and doses log₁₀1 to log₁₀5 of the strain C5. The results are presented in the following table. | | |

| **Strain** | **oral LD**_{**50**} **(log**_{**10**} **cfu)** | **i.v.LD**_{**50**} **(log**_{**10**} **cfu)** |
|---|---|---|
| C5 | 4.16 | <1.87 |
| BRD 441 | 8.62 | 2.46 |
| BRD1115 | 8.98 | 5.22 |

**Table 3:**

| | | | |
|---|---|---|---|
| The ability of the defined *surA* mutant strain to confer protection against homologous challenge with the wild type strain C5 was determined. Groups of 5 BALB/c mice were orally immunised with log₁₀8 organisms of the strain BRD1115 then challenged with log₁₀4 to log₁₀10 of the mouse virulent strain C5 either 4 or 10 weeks post inoculation. The new LD₅₀ was then calculated and the results presented in the table below. | | | |

| **Immunising strain** | **oral LD**_{**50**} **of C5** | | **protection (no of LD**_{**50**}**'s)** |
|---|---|---|---|
| | 4 weeks post immunisation | 10 weeks post immunisation | |
| BRD1115 | 8.58 | | -3800 |
| none | 4.74 | | |
| | | | |
| BRD 1115 | | 9.51 | ∼4800 |
| none | | 4.68 | |

**Table 4:**

| | |
|---|---|
| Three groups of 10 mice were immunised with the strains BRD1115, BRD1126 and BRD 1127 and then challenged 4 weeks post immunisation with 50 LD₅₀ doses of tetanus toxin subcutaneously. Deaths were noted over 4 days. The numbers of mice surviving the challenge are presented in the table below. | |

| **Strain** | **Survivors after challenge** |
|---|---|
| BRD 1115 | 0/10 |
| BRD 1126 (*nirB*) | 6/10 |
| BRD 1127 (*htrA*) | 10/10 |

**Table 5:**

| | | | | | | | |
|---|---|---|---|---|---|---|---|
| The numbers of bacteria (cfu) present in the cultures of the complemented strain K2 following culture in L broth with and without the antibiotic kanamycin were calculated. The cultures were then plated onto L agar with and without kanamycin to show presence of the plasmid pLG339/*surA*. The results are presented as a total number and also the kanamycin resistant colonies as a percentage of the total bacteria present. | | | | | | | |

| **Kanamycin Kanamycin** | | **numbers of bacteria (cfu/ml)** | | | | | |
|---|---|---|---|---|---|---|---|
| **in broth** | **in agar** | **30 hours** | **(%)** | **48 hours** | **(%)** | **72 hours** | **(%)** |
| ++ | ++ | 4.75x10⁷ | (95%) | 6x10⁷ | (71%) | 8.25x10⁷ | (82.5%) |
| ++ | -- | 5 x10⁷ | | 8.5x10⁷ | | 10x10⁷ | |
| -- | ++ | 5.25x10⁷ | (124%) | 9.5x10⁷ | (111%) | 6x10⁷ | (89%) |
| -- | -- | 4.25x10⁷ | | 8.5x10⁷ | | 6.75x10⁷ | |

### References

- 1: Bacon; G.A., Burrows, T. W. and Yates, M. (1950) Br. J. Exp. Pathol.., 31, 714-24.
- 2.: Chatfield, S.N., Charles, I.G., Makoff, A.J. et. al. (1992a) Biotech, 10, 888-892.
- 3.: Chatfield, S.N. Strahan,K., Pickard, D., Charles, I.G., Hormaeche, C.E. and Dougan, G. ( 1992b) Microbiol. Pathog. , 12, 145-151.
- 4.: Chatfield, S.N. Fairweather, N., Charles, I., Pickard, D., Levine, M. Hone, D., Posanda, M., Strugnell, R.A. and Dougan G. (1992) Vaccine, 10, 53-60.
- 5.: Curtiss III, R. and Kelly, S.M. (1987) Infect. Immun. 55, 3035-3043.
- 6.: Dougan, G. Chatfield, S., Pickard, D., Bester, J., O'Callaghan, D. and Maskell, D. (1988) J. Inf. Dis, 158,1329-1335.
- 7.: Fairweather N.F., Lyness V.A., and Maskell D.J., (1987) Infect. Immun. 55, 2541-2545
- 8.: Fairweather, N.F., Chatfield, S.N. Makoff, A.J. et. al. (1990) Infect. Immun., 58, 1323-1329.
- 9.: Gomaz-Duarte, O.G., Galen, J. Chatfield, et. al. (1995) Vaccine, 13:1596-1602.
- 10.: Harrison J.A., Pickard D., Higgins C.F., Khan A., Chatfield S., Ali T., Dorman C.J. Hormaeche C., and Dougan G., (1994) Mol. Micro., 13, 133-140
- 11.: Hohmann, E.L., Oletta, C.A., Killeen, K.P. and Miller, S.I. (1996) Vaccine 14, 19-24.
- 12.: Hone, D., Morona, R., Attridge, S. and Hackett, J. (1987) J. Infect. Dis., 156, 167-1
- 13.: Hull R.A. Gill R.E. Hsu P., Minshew B.H., and Falkow S., (1981) Infect. Immun. 33,933-938
- 14.: Johnson K., Charles I., Dougan G., Pickard D., O'Gaora P., Costa G., Ali T., Miller I., and Hormaeche C. (1991) Mol. Micro., 5, 401-407
- 15.: Jones, P.W., Dougan, G. Haywood, C., .MacKensie, N., Collins, P. and Chatfield, S.N. (1991) Vaccine 9, 29-36.
- 16.: Lazar S.W., and Kolter R., (1996) J.Bact. 178, 1770-1773
- 17.: Levine, M. M., Galen, J., Barry, E., et al (1995) J. Biotech., 44, 193-196.
- 18.: Manoil, C. and Beckwith, J. (1985) Proc. Natl. Acad. Sci., USA 82, 8129-8133.
- 19.: Miles, A.A., Misra, S.S. and Irwin, J. (1938) J. Hygiene, 38, 732-749.
- 20.: Miller I., Chatfield S., Dougan G., Desilva L., Joysey H.S., and Hormaeche
- C.,: (1989a) Mol. Gen. Genet., 215, 312-316
- 21.: Miller, I., Maskell, D., Hormaeche, C., Pickard, D. and Dougan, G. (1989b) Infect. Immun. 57,2758-2763.
- 22.: Miller, S.I., Kukral, A.M. and Mekalanos, J.J. (1989). Proc. Natl. Acad. Sci., USA 86, 5054-5058.
- 23.: Miller V.L., and Mekalanos J.J. (1988) J.Bact. 170, 2575
- 24.: Missiakis D., Betton J.M., and Raina S., (1996) Mol. Micro., 21, 871-884
- 25.: Oxer, M.D., Bentley, C.M., Doyle, J.G. Peakman, T.C., Charles, I.G. and Makoff, A.J. (1991) Nucl. Acids Res. 19, 2889-2892.
- 26.: Pickard, D., Li., J.L., Roberts, M., Maskell, D., Hone, D., Levine, M., Dougan, G. and Chatfield, S. (1994), 62, 3984-3993.
- 27.: Reed L.J., and Meunch H., (1938) Am. J. Hygiene 27, 493-497
- 28.: Roberts M., Bacon A., Rappuoli R., Pizza M., Cropley I., Douce G., Dougan G., 27 Marinaro M., McGhee J., and Chatfield S., (1995) Infect. Immun. 63, 2100-2108
- 29.: Rouviere P.E., and Gross C.A., (1996) Genes Dev., 10, 3170-3182
- 30.: Rudd K.E., Sofia H.J., Koonin E.V., Plunkett III G., Lazar S., and Rouviere P.E. (1995) TIBS 20, 12-14.
- 31.: Sambrook J., Fritsch E.F., and Maniatis T., (1989) Molecular Cloning. A Laboratory Manual. Cold Spring Harbor Laboratory, Cold Spring Harbor, NY, USA
- 32.: Strugnell, R.A. Dougan, G., Chatfied, S.N. et. al. (1992) Infect. Immun., 60, 3994-4002.
- 33.: Tormo A., Almiron M., and Kolter R., (1990) J.Bact. 172, 4339-4347
- 34.: Yura T., Mori H., Nagai H., Nagata T., Ishihama A., Fujita N., Isono K., Mizobuchi K., and Nakata A.(1992) Nucl. Acids Res., 20, 3305-3308
- 35.: EP-B-0322237 (Dougan et al)
- 36.: EP-B-0400958 (Dougan et al)
- 37.: EP-B-0524205 (Dougan et al)
- 38.: WO 92/15689 (Charles et al)
- 39.: Chatfield, S.N., Strugnell. R.A. and Dougan, G (1989) Vaccine, 7, 495-498
- 40.: Everest, P., Allen, J., Papakonstantinopoulou, A., Mastroeni, P., Roberts, M. and Dougan, G. (1995) FEMS Microbiol. Letts., 126, 97-101
- 41.: Stoker N.G., Fairweather N.F., and Spratt B.G. (1982) Gene 18(3) 335-341

## Claims

1. A vaccine comprising a pharmaceutically acceptable carrier or diluent and a bacterium attenuated by a non-reverting mutation in a gene encoding a protein which promotes folding of extracytoplasmic proteins.

2. A vaccine according to claim 1 wherein the protein encoded by the mutant gene is a periplasmic protein.

3. A vaccine according to claim 1 or 2 wherein the protein encoded by the mutant gene promotes the folding of secreted proteins.

4. A vaccine according to claim 1, 2 or 3 wherein the protein encoded by the mutant gene is a peptidyl-prolyl cis-trans isomerase (PPiase).

5. A vaccine according to claim 4 wherein the PPiase is a member of the parvulin family of PPiases.

6. A vaccine according to any one of the preceding claims wherein the protein encoded by the mutant gene is SurA.

7. A vaccine according to any one of the preceding claims wherein the bacterium is further attenuated by a non-reverting mutation in a second gene.

8. A vaccine according to claim 7 wherein the second gene is an *aro* gene, a *pur* gene, the *htrA* gene, the *ompR* gene, the *galE* gene, the *cya* gene, the *crp* gene or the *phoP* gene.

9. A vaccine according to claim 8 wherein the *aro* gene is *aroA*, *aroC*, *aroD* or *aroE*.

10. A vaccine according to any one of the preceding claims wherein the mutation in the gene encoding a protein which promotes folding of extracytoplasmic proteins and/or the mutation in the second gene is a defined mutation.

11. A vaccine according to any one of the preceding claims wherein the bacterium has no uncharacterised mutations in the genome thereof.

12. A vaccine according to any one of the preceding claims wherein the bacterium is a bacterium that infects via the oral route.

13. A vaccine according to any one of the preceding claims wherein the bacterium is from the genera Salmonella, Escherichia, Vibrio, Haemophilus, Neisseria, Yersinia, Bordetella or Brucella.

14. A vaccine according to claim 13 wherein the bacterium is *Salmonella typhimurium*, *Salmonella typhi*, *Salmonella enteritidis*, *Salmonella choleraesuis*, *Salmonella dublin*, *Escherichia coli*, *Haemophilus influenzae*, *Neisseria gonorrhoeae*, *Yersinia enterocolitica*, *Bordetella pertussis* or *Brucella abortus*.

15. A vaccine according to any one of the preceding claims wherein the bacterium is genetically engineered to express an antigen from another organism.

16. A vaccine according to claim 15 wherein the antigen is fragment C of tetanus toxin.

17. A vaccine according to claim 15 or 16 wherein expression of the antigen is driven by the *nirB* promoter or the *htrA* promoter.

18. A bacterium attenuated by a non-reverting mutation as defined in any one of the preceding claims for use in a method of vaccinating a human or animal.

19. Use of a bacterium as defined in any one of the preceding claims for the manufacture of a medicament for vaccinating a human or animal.

## Patentansprüche

1. Impfstoff, umfassend einen pharmazeutisch geeigneten Träger oder Verdünnungsmittel und ein Bakterium, das durch eine nicht-reversible Mutation in einem Gen abgeschwächt ist, das für ein Protein kodiert, welches die Faltung von extracytoplasmatischen Proteinen fördert.

2. Impfstoff nach Anspruch 1, worin das Protein, das durch das mutante Gen kodiert ist, ein periplasmatisches Protein ist.

3. Impfstoff nach Anspruch 1 oder 2, worin das Protein, das durch das mutante Gen kodiert ist, die Faltung von sekretierten Proteinen fördert.

4. Impfstoff nach Anspruch 1, 2 oder 3, worin das Protein, das durch das mutante Gen kodiert ist, eine Peptidyl-Prolyl-cis-trans-Isomerase (PPiase) ist.

5. Impfstoff nach Anspruch 4, worin die PPiase ein Mitglied der Parvulin-Familie der PPiasen ist.

6. Impfstoff nach einem der vorangehenden Ansprüche, worin das Protein, das durch das mutante Gen kodiert ist, SurA ist.

7. Impfstoff nach einem der vorangehenden Ansprüche, worin das Bakterium außerdem durch eine nicht-reversible Mutation in einem zweiten Gen abgeschwächt ist.

8. Impfstoff nach Anspruch 7, worin das zweite Gen ein *aro*-Gen, ein *pur*-Gen, das *htrA*-Gen, das *ompR*-Gen, das *galE*-Gen, das *cya*-Gen, das *crp*-Gen oder das *phoP*-Gen ist.

9. Impfstoff nach Anspruch 8, worin das *aro*-Gen *aroA, aroC, aroD* oder *aroE* ist.

10. Impfstoff nach einem der vorangehenden Ansprüche, worin die Mutation in dem Gen, das für ein Protein kodiert, welches die Faltung von extracytoplasmatischen Proteinen und/oder die Mutation in dem zweiten Gen fördert, eine definierte Mutation ist.

11. Impfstoff nach einem der vorangehenden Ansprüche, worin das Bakterium keine uncharakterisierten Mutationen in seinem Genom aufweist.

12. Impfstoff nach einem der vorangehenden Ansprüche, worin das Bakterium ein Bakterium ist, welches über den oralen Weg infiziert.

13. Impfstoff nach einem der vorangehenden Ansprüche, worin das Bakterium von den Gattungen Salmonella, Escherichia, Vibrio, Haemophilus, Neisseria, Yersinia, Bordetalla oder Brucella stammt.

14. Impfstoff nach Anspruch 13, worin das Bakterium *Salmonella typhimurium, Salmonella typhi, Salmonella enteritidis, Salmonella choleraesuis, Salmonella dublin*, *Escherichia coli, Haemophilus influenzae, Neisseria gonorrhoeae, Yersinia enterocolitica, Bordetella pertussis* oder *Brucella abortus* ist.

15. Impfstoff nach einem der vorangehenden Ansprüche, worin das Bakterium gentechnisch verändert ist zum Exprimieren eines Antigens aus einem anderen Organismus.

16. Impfstoff nach Anspruch 15, wobei das Antigen Fragment C von Tetanus-Toxin ist.

17. Impfstoff nach Anspruch 15 oder 16, worin die Expression des Antigens durch den *nirB*-Promotor oder den *htrA*-Promotor vorangetrieben wird.

18. Bakterium, abgeschwächt durch eine nicht-reversible Mutation, wie in einem der vorangehenden Ansprüche definiert, zur Verwendung bei einer Methode zum Impfen eines Menschen oder Tiers.

19. Verwendung eines Bakteriums, wie in einem der vorangehenden Ansprüche definiert, für die Herstellung eines Medikaments zum Impfen eines Menschen oder Tiers.

## Revendications

1. Vaccin comprenant un diluant ou un véhicule pharmaceutiquement acceptable et une bactérie atténuée par une mutation non réversible dans un gène codant pour une protéine qui favorise le repliement de protéines extra-cytoplasmiques.

2. vaccin selon la revendication 1 dans lequel la protéine codée par le gène mutant est une protéine périplasmique.

3. Vaccin selon la revendication 1 ou 2 dans lequel la protéine codée par le gène mutant favorise le repliement de protéines sécrétées.

4. Vaccin selon la revendication 1, 2 ou 3, dans lequel la protéine codée par le gène mutant est une peptidyl-prolyl cis-trans isomérase (PPiase).

5. Vaccin selon la revendication 4, dans lequel la PPiase est un membre de la famille des parvulines de PPiases.

6. Vaccin selon l'une quelconque des revendications précédentes dans lequel la protéine codée par le gène mutant est SurA.

7. Vaccin selon l'une quelconque des revendications précédentes dans lequel la bactérie est en outre atténuée par une mutation non réversible dans un second gène.

8. Vaccin selon la revendication 7 dans lequel le second gène est un gène *aro*, un gène *pur*, le gène *htrA*, le gène *ompR*, le gène *galE*, le gène *cya*, le gène *crp* ou le gène *phoP*.

9. Vaccin selon la revendication 8 dans lequel le gène *aro* est *aroA, aroC, aroD* ou *aroE*.

10. Vaccin selon l'une quelconque des revendications précédentes dans lequel la mutation dans le gène codant pour une protéine qui favorise le repliement de protéines extra-cytoplasmiques et/ou la mutation dans le second gène est une mutation définie.

11. Vaccin selon l'une quelconque des revendications précédentes dans lequel la bactérie n'a pas de mutations non caractérisées dans son génome.

12. Vaccin selon l'une quelconque des revendications précédentes dans lequel la bactérie est une bactérie qui infecte via la voie orale.

13. Vaccin selon l'une quelconque des revendications précédentes dans lequel la bactérie appartenant aux genres Salmonella, Escherichia, Vibrio, Haemophilus, Neisseria, Yersinia, Bordetella ou Brucella.

14. Vaccin selon la revendication 13, dans lequel la bactérie est *Salmonella typhimurium, Salmonella ty* *phi, Salmonella enteritidis*, *Salmonella choleraesuis*, *Salmonella dublin, Escherichia coli, Haemophilus influenzae, Neisseria gonorrhoeae, Yersinia enterocolitica, Bordetella pertussis* ou *Brucella abortus*.

15. Vaccin selon l'une quelconque des revendications précédentes dans lequel la bactérie est génétiquement manipulée pour exprimer un antigène d'un autre organisme.

16. Vaccin selon la revendication 15 dans lequel l'antigène est le fragment C de la toxine du tétanos.

17. Vaccin selon la revendication 15 ou 16 dans lequel l'expression de l'antigène est commandée par le promoteur *nirB* ou le promoteur *htrA*.

18. Bactérie atténuée par une mutation non réversible telle que définie dans l'une quelconque des revendications précédentes pour utilisation dans une méthode de vaccination d'un humain ou d'un animal.

19. Utilisation d'une bactérie telle que définie dans l'une quelconque des revendications précédentes pour la fabrication d'un médicament pour la vaccination d'un humain ou d'un animal.


(1) GENERAL INFORMATION:
   (i) APPLICANT:
      (A) NAME: Medeva Europe Limited
      (B) STREET: 10 St James's Street
      (C) CITY: London
      (D) STATE: not applicable
      (E) COUNTRY: United Kingdom
      (F) POSTAL CODE (ZIP): SW1A 1EF
   (ii) TITLE OF INVENTION: VACCINES CONTAINING ATTENUATED BACTERIA
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS.
      (D) SOFTWARE: PatentIn Release #1.0. Version #1.30 (EPO)
(2) INFORMATION FOR SEQ ID NO: 1:
   (i) SEQUENCE CHARACTERISTICS:
      (A) LENGTH: 1287 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Salmonella typhimurium
   (ix) FEATURE:
      (A) NAME/KEY: CDS
      (B) LOCATION:1..1281
   (xi) SEQUENCE DESCRIPTION: SEQ ID NO: 1:
   ATG AAG AAC TGG AAA ACG CTG CTT CTC GGT ATC GCC ATG ATC GCG AAT    48
   Met Lys Asn Trp Lys Thr Leu Leu Leu Gly Ile Ala Met Ile Ala Asn
    1               5                  10                  15
   ACC AGT TTC GCT GCC CCC CAG GTA GTC GAT AAA GTC GCA GCC GTC GTC    96
   Thr Ser Phe Ala Ala Pro Gln Val Val Asp Lys Val Ala Ala Val Val
               20                  25                  30
   AAT AAT GGC GTC GTG CTG GAA AGC GAC GTT GAT GGC TTA ATG CAA TCA   144
   Asn Asn Gly Val Val Leu Glu Ser Asp Val Asp Gly Leu Met Gln Ser
           35                  40                  45
   GTC AAA CTC AAC GCG GGT CAG GCA GGT CAG CAG CTT CCG GAC GAC GCC     192
   Val Lys Leu Asn Ala Gly Gln Ala Gly Gln Gln Leu Pro Asp Asp Ala
        50                  55                  60
   ACG CTG CGT CAC CAG ATC CTG GAA CGT TTG ATT ATG GAT CAA ATT ATC     240
   Thr Leu Arg His Gln Ile Leu Glu Arg Leu Ile Met Asp Gln Ile Ile
    65                  70                  75                  80
   CTG CAG ATG GGT CAG AAG ATG GGG GTG AAG ATC ACG GAT GAG CAG TTG     288
   Leu Gln Met Gly Gln Lys Met Gly Val Lys Ile Thr Asp Glu Gln Leu
                    85                  90                  95
   GAT CAG CCA TCA GCC AAC ATC GCC AAA CAA AAC AAT ATG ACG ATG GAT     336
   Asp Gln Pro Ser Ala Asn Ile Ala Lys Gln Asn Asn Met Thr Met Asp
               100                 105                 110
   CAG ATG CGC AGC CGT CTG GCT TAC GAT GGG CTG AAC TAT TCA ACC TAC     384
   Gln Met Arg Ser Arg Leu Ala Tyr Asp Gly Leu Asn Tyr Ser Thr Tyr
           115                 120                 125
   CGT AAC CAG ATT CGT AAA GAG ATG ATT ATC TCT GAA GTG CGC AAC AAT     432
   Arg Asn Gln Ile Arg Lys Glu Met Ile Ile Ser Glu Val Arg Asn Asn
       130                 135                 140
   GAG GTT CGT CGC CGT ATC ACC GTT TTG CCG CAA GAA GTT GAC GCG CTG     480
   Glu Val Arg Arg Arg Ile Thr Val Leu Pro Gln Glu Val Asp Ala Leu
   145                 150                 155                 160
   GCA AAA CAG ATT GGC ACC CAA AAC GAT GCC AGC ACC GAG CTG AAC CTG     528
   Ala Lys Gln Ile Gly Thr Gln Asn Asp Ala Ser Thr Glu Leu Asn Leu
                   165                 170                 175
   AGC CAT ATC CTG ATT GCT CTG CCG GAA AAC CCA ACC TCC GAG CAG GTT     576
   Ser His Ile Leu Ile Ala Leu Pro Glu Asn Pro Thr Ser Glu Gln Val
               180                 185                 190
   AAC GAC GCG CAG CGC CAG GCG GAA AGC ATT GTT GAA GAA GCG CGT AAC     624
   Asn Asp Ala Gln Arg Gln Ala Glu Ser Ile Val Glu Glu Ala Arg Asn
           195                 200                 205
   GGC GCA GAT TTC GGC AAA CTG GCG ATT ACC TAC TCT GCC GAC CAG CAG     672
   Gly Ala Asp Phe Gly Lys Leu Ala Ile Thr Tyr Ser Ala Asp Gln Gln
       210                 215                 220
   GCG CTA AAA GGC GGT CAG ATG GGC TGG GGC CGT ATC CAG GAG CTG CCG     720
   Ala Leu Lys Gly Gly Gln Met Gly Trp Gly Arg Ile Gln Glu Leu Pro
   225                 230                 235                 240
   GGG ATT TTC GCC CAG GCG CTG AGC ACC GCG AAG AAA GGC GAC ATT GTC     768
   Gly Ile Phe Ala Gln Ala Leu Ser Thr Ala Lys Lys Gly Asp Ile Val
                   245                 250                 255
   GGC CCG ATT CGC TCC GGC GTC GGC TTC CAC ATT CTG AAA GTA AAT GAC     816
   Gly Pro Ile Arg Ser Gly Val Gly Phe His Ile Leu Lys Val Asn Asp
               260                 265                 270
   CTG CGC GGT CAG AGC CAG AGT ATC TCC GTG ACC GAA GTT CAC GCT CGT         864
   Leu Arg Gly Gln Ser Gln Ser Ile Ser Val Thr Glu Val His Ala Arg
           275                 280                 285
   CAC ATT CTG CTT AAG CCG TCG CCG ATC ATG AAC GAT CAG CAG GCG CGC         912
   His Ile Leu Leu Lys Pro Ser Pro Ile Met Asn Asp Gln Gln Ala Arg
       290                 295                 300
   CTG AAG CTG GAA GAA ATC GCG GCT GAC ATT AAG AGT GGT AAA ACC ACC         960
   Leu Lys Leu Glu Glu Ile Ala Ala Asp Ile Lys Ser Gly Lys Thr Thr
   305                 310                 315                 320
   TTT GCC GCT GCG GCG AAA GAG TAC TCT CAG GAC CCG GGC TCC GCT AAC        1008
   Phe Ala Ala Ala Ala Lys Glu Tyr Ser Gln Asp Pro Gly Ser Ala Asn
                   325                 330                 335
   CAG GGC GGT GAT TTG GGT TGG GCT ACG CCA GAT ATT TTC GAC CCG GCG        1056
   Gln Gly Gly Asp Leu Gly Trp Ala Thr Pro Asp Ile Phe Asp Pro Ala
               340                 345                 350
   TTC CGC GAC GCG CTA ACG AAG CTG CAT AAA GGC CAA ATA AGC GCG CCG        1104
   Phe Arg Asp Ala Leu Thr Lys Leu His Lys Gly Gln Ile Ser Ala Pro
           355                 360                 365
   GTA CAC TCC TCT TTC GGC TGG CAT CTG ATC GAA TTG CTG GAT ACG CGT        1152
   Val His Ser Ser Phe Gly Trp His Leu Ile Glu Leu Leu Asp Thr Arg
       370                 375                 380
   AAG GTA GAC AAA ACC GAT GCG GCG CAG AAA GAT CGC GCT TAT CGT ATG        1200
   Lys Val Asp Lys Thr Asp Ala Ala Gln Lys Asp Arg Ala Tyr Arg Met
   385                 390                 395                 400
   CTG ATG AAC CGT AAA TTC TCA GAA GAA GCG GCG ACC TGG ATG CAA GAA        1248
   Leu Met Asn Arg Lys Phe Ser Glu Glu Ala Ala Thr Trp Met Gln Glu
                   405                 410                 415
   CAG CGC GCC ACT TAC GTT AAG ATT TTG AGT AAC TAATGA                     1287
   Gln Arg Ala Thr Tyr Val Lys Ile Leu Ser Asn
               420                 425
(2) INFORMATION FOR SEQ ID NO: 2:
   (i) SEQUENCE CHARACTERISTICS:
      (A) LENGTH: 427 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Lys Asn Trp Lys Thr Leu Leu Leu Gly Ile Ala Met Ile Ala Asn
   1 5 10 15
   Thr Ser Phe Ala Ala Pro Gln Val Val Asp Lys Val Ala Ala Val Val
   20 25 30
   Asn Asn Gly Val Val Leu Glu Ser Asp Val Asp Gly Leu Met Gln Ser
   35 40 45
   Val Lys Leu Asn Ala Gly Gln Ala Gly Gln Gln Leu Pro Asp Asp Ala
   50 55 60
   Thr Leu Arg His Gln Ile Leu Glu Arg Leu Ile Met Asp Gln Ile Ile
   65 70 75 80
   Leu Gln Met Gly Gln Lys Met Gly Val Lys Ile Thr Asp Glu Gln Leu
   85 90 95
   Asp Gln Pro Ser Ala Asn Ile Ala Lys Gln Asn Asn Met Thr Met Asp
   100 105 110
   Gln Met Arg Ser Arg Leu Ala Tyr Asp Gly Leu Asn Tyr Ser Thr Tyr
   115 120 125
   Arg Asn Gln Ile Arg Lys Glu Met Ile Ile Ser Glu Val Arg Asn Asn
   130 135 140
   Glu Val Arg Arg Arg Ile Thr Val Leu Pro Gln Glu Val Asp Ala Leu
   145 150 155 160
   Ala Lys Gln Ile Gly Thr Gln Asn Asp Ala Ser Thr Glu Leu Asn Leu
   165 170 175
   Ser His Ile Leu Ile Ala Leu Pro Glu Asn Pro Thr Ser Glu Gln Val
   180 185 190
   Asn Asp Ala Gln Arg Gln Ala Glu Ser Ile Val Glu Glu Ala Arg Asn
   195 200 205
   Gly Ala Asp Phe Gly Lys Leu Ala Ile Thr Tyr Ser Ala Asp Gln Gln
   210 215 220
   Ala Leu Lys Gly Gly Gln Met Gly Trp Gly Arg Ile Gln Glu Leu Pro
   225 230 235 240
   Gly Ile Phe Ala Gln Ala Leu Ser Thr Ala Lys Lys Gly Asp Ile Val
   245 250 255
   Gly Pro Ile Arg Ser Gly Val Gly Phe His Ile Leu Lys Val Asn Asp
   260 265 270
   Leu Arg Gly Gln Ser Gln Ser Ile Ser Val Thr Glu Val His Ala Arg
   275 280 285
   His Ile Leu Leu Lys Pro Ser Pro Ile Met Asn Asp Gln Gln Ala Arg
   290 295 300
   Leu Lys Leu Glu Glu Ile Ala Ala Asp Ile Lys Ser Gly Lys Thr Thr
   305 310 315 320
   Phe Ala Ala Ala Ala Lys Glu Tyr Ser Gln Asp Pro Gly Ser Ala Asn
   325 330 335
   Gln Gly Gly Asp Leu Gly Trp Ala Thr Pro Asp Ile Phe Asp Pro Ala
   340 345 350
   Phe Arg Asp Ala Leu Thr Lys Leu His Lys Gly Gln Ile Ser Ala Pro
   355 360 365
   Val His Ser Ser Phe Gly Trp His Leu Ile Glu Leu Leu Asp Thr Arg
   370 375 380
   Lys Val Asp Lys Thr Asp Ala Ala Gln Lys Asp Arg Ala Tyr Arg Met
   385 390 395 400
   Leu Met Asn Arg Lys Phe Ser Glu Glu Ala Ala Thr Trp Met Gln Glu
   405 410 415
   Gln Arg Ala Thr Tyr Val Lys Ile Leu Ser Asn
   420 425
(2) INFORMATION FOR SEQ ID NO: 3:
   (i) SEQUENCE CHARACTERISTICS:
      (A) LENGTH: 1287 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: E.coli
   (ix) FEATURE:
      (A) NAME/KEY: CDS
      (B) LOCATION:1..1284
   (xi) SEQUENCE DESCRIPTION: SEQ ID NO: 3:
   ATG AAG AAC TGG AAA ACG CTG CTT CTC GGT ATC GCC ATG ATC GCG AAT      48
   Met Lys Asn Trp Lys Thr Leu Leu Leu Gly Ile Ala Met Ile Ala Asn
           430                 435                 440
   ACC AGT TTC GCT GCC CCC CAG GTA GTC GAT AAA GTC GCA GCC GTC GTC      96
   Thr Ser Phe Ala Ala Pro Gln Val Val Asp Lys Val Ala Ala Val Val
       445                 450                 455
   AAT AAC GGC GTC GTG CTG GAA AGC GAC GTT GAT GGA TTA ATG CAG TCG     144
   Asn Asn Gly Val Val Leu Glu Ser Asp Val Asp Gly Leu Met Gln Ser
   460                 465                 470                 475
   GTA AAA CTG AAC GCT GCT CAG GCA AGG CAG CAA CTT CCT GAT GAC GCG     192
   Val Lys Leu Asn Ala Ala Gln Ala Arg Gln Gln Leu Pro Asp Asp Ala
                480                     485                     490
   ACG CTG CGC CAC CAA ATC ATG GAA CGT TTG ATC ATG GAT CAA ATC ATT      240
   Thr Leu Arg His Gln Ile Met Glu Arg Leu Ile Met Asp Gln Ile Ile
               495                     500                     505
   CTG CAG ATG GGG CAG AAA ATG GGA GTG AAA ATC TCC GAT GAG CAG CTG      288
   Leu Gln Met Gly Gln Lys Met Gly Val Lys Ile Ser Asp Glu Gln Leu
           510                     515                     520
   GAT CAG GCG ATT GCT AAC ATC GCG AAA CAG AAC AAC ATG ACG CTG GAT      336
   Asp Gln Ala Ile Ala Asn Ile Ala Lys Gln Asn Asn Met Thr Leu Asp
       525                     530                     535
   CAG ATG CGC AGC CGT CTG GCT TAC GAT GGA CTG AAC TAC AAC ACC TAT      384
   Gln Met Arg Ser Arg Leu Ala Tyr Asp Gly Leu Asn Tyr Asn Thr Tyr
   540                     545                     550                     555
   CGT AAC CAG ATC CGC AAA GAG ATG ATT ATC TCT GAA GTG CGT AAC AAC      432
   Arg Asn Gln Ile Arg Lys Glu Met Ile Ile Ser Glu Val Arg Asn Asn
                   560                     565                     570
   GAG GTG CGT CGT CGC ATC ACC ATC CTG CCG CAG GAA GTC GAA TCC CTG      480
   Glu Val Arg Arg Arg Ile Thr Ile Leu Pro Gln Glu Val Glu Ser Leu
               575                     580                     585
   GCG CAG CAG GTG GGT AAC CAA AAC GAC GCC AGC ACT GAG CTG AAC CTG      528
   Ala Gln Gln Val Gly Asn Gln Asn Asp Ala Ser Thr Glu Leu Asn Leu
           590                     595                     600
   AGC CAC ATC CTG ATC CCG CTG CCG GAA AAC CCG ACC TCT GAT CAG GTG      576
   Ser His Ile Leu Ile Pro Leu Pro Glu Asn Pro Thr Ser Asp Gln Val
       605                     610                     615
   AAC GAA GCG GAA AGC CAG GCG CGC GCC ATT GTC GAT CAG GCG CGT AAC      624
   Asn Glu Ala Glu Ser Gln Ala Arg Ala Ile Val Asp Gln Ala Arg Asn
   620                     625                     630                     635
   GGC GCT GAT TTC GGT AAG CTG GCG ATT GCT CAT TCT GCC GAC CAG CAG      672
   Gly Ala Asp Phe Gly Lys Leu Ala Ile Ala His Ser Ala Asp Gln Gln
                   640                     645                     650
   GCG CTG AAC GGC GGC CAG ATG GGC TGG GGC CGT ATT CAG GAG TTG CCC      720
   Ala Leu Asn Gly Gly Gln Met Gly Trp Gly Arg Ile Gln Glu Leu Pro
               655                     660                     665
   GGG ATC TTC GCC CAG GCA TTA AGC ACC GCG AAG AAA GGC GAC ATT GTT      768
   Gly Ile Phe Ala Gln Ala Leu Ser Thr Ala Lys Lys Gly Asp Ile Val
           670                     675                     680
   GGC CCG ATT CGT TCC GGC GTT GGC TTC CAT ATT CTG AAA GTT AAC GAC      816
   Gly Pro Ile Arg Ser Gly Val Gly Phe His Ile Leu Lys Val Asn Asp
       685                     690                     695
   CTG CGC GGC GAA AGC AAA AAT ATC TCG GTG ACC GAA GTT CAT GCT CGC      864
   Leu Arg Gly Glu Ser Lys Asn Ile Ser Val Thr Glu Val His Ala Arg
   700                 705                 710                 715
   CAT ATT CTG CTG AAA CCG TCG CCG ATC ATG ACT GAC GAA CAG GCC CGT      912
   His Ile Leu Leu Lys Pro Ser Pro Ile Met Thr Asp Glu Gln Ala Arg
                   720                 725                 730
   GTG AAA CTG GAA CAG ATT GCT GCT GAT ATC GAG AGT GGT AAA ACG ACT      960
   Val Lys Leu Glu Gln Ile Ala Ala Asp Ile Glu Ser Gly Lys Thr Thr
               735                 740                 745
   TTT GCT GCC GCA ACG AAA GAG TTC TCT CAG GAT CCA GTC TCT GCT AAC     1008
   Phe Ala Ala Ala Thr Lys Glu Phe Ser Gln Asp Pro Val Ser Ala Asn
           750                 755                 760
   CAG GGC GGC GAT CTC GGC TGG GCT ACA CCA GAT ATT TTC GAT CCG GCC     1056
   Gln Gly Gly Asp Leu Gly Trp Ala Thr Pro Asp Ile Phe Asp Pro Ala
       765                 770                 775
   TTC CGT GAC GCC CTG ACT CGC CTG AAC AAA GGT CAA ATG AGT GCA CCG     1104
   Phe Arg Asp Ala Leu Thr Arg Leu Asn Lys Gly Gln Met Ser Ala Pro
   780                 785                 790                 795
   GTT CAC TCT TCA TTC GGC TGG CAT TTA ATC GAA CTG CTG GAT ACC CGT     1152
   Val His Ser Ser Phe Gly Trp His Leu Ile Glu Leu Leu Asp Thr Arg
                   800                 805                 810
   AAT GTC GAT AAA ACC GAC GCT GCG CAG AAA GAT CGT GCA TAC CGC ATG     1200
   Asn Val Asp Lys Thr Asp Ala Ala Gln Lys Asp Arg Ala Tyr Arg Met
               815                 820                 825
   CTG ATG AAC CGT AAG TTC TCG GAA GAA GCA GCA AGC TGG ATG CAG GAA     1248
   Leu Met Asn Arg Lys Phe Ser Glu Glu Ala Ala Ser Trp Met Gln Glu
           830                 835                 840
   CAA CGT GCC AGC GCC TAC GTT AAA ATC CTG AGC AAC TAA                 1287
   Gln Arg Ala Ser Ala Tyr Val Lys Ile Leu Ser Asn
       845                 850                 855
(2) INFORMATION FOR SEQ ID NO: 4:
   (i) SEQUENCE CHARACTERISTICS:
      (A) LENGTH: 428 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Met Lys Asn Trp Lys Thr Leu Leu Leu Gly Ile Ala Met Ile Ala Asn
   1 5 10 15
   Thr Ser Phe Ala Ala Pro Gln Val Val Asp Lys Val Ala Ala Val Val
   20 25 30
   Asn Asn Gly Val Val Leu Glu Ser Asp Val Asp Gly Leu Met Gln Ser
   35 40 45
   Val Lys Leu Asn Ala Ala Gln Ala Arg Gln Gln Leu Pro Asp Asp Ala
   50 55 60
   Thr Leu Arg His Gln Ile Met Glu Arg Leu Ile Met Asp Gln Ile Ile
   65 70 75 80
   Leu Gln Met Gly Gln Lys Met Gly Val Lys Ile Ser Asp Glu Gln Leu
   85 90 95
   Asp Gln Ala Ile Ala Asn Ile Ala Lys Gln Asn Asn Met Thr Leu Asp
   100 105 110
   Gln Met Arg Ser Arg Leu Ala Tyr Asp Gly Leu Asn Tyr Asn Thr Tyr
   115 120 125
   Arg Asn Gln Ile Arg Lys Glu Met Ile Ile Ser Glu Val Arg Asn Asn
   130 135 140
   Glu Val Arg Arg Arg Ile Thr Ile Leu Pro Gln Glu Val Glu Ser Leu
   145 150 155 160
   Ala Gln Gln Val Gly Asn Gln Asn Asp Ala Ser Thr Glu Leu Asn Leu
   165 170 175
   Ser His Ile Leu Ile Pro Leu Pro Glu Asn Pro Thr Ser Asp Gln Val
   180 185 190
   Asn Glu Ala Glu Ser Gln Ala Arg Ala Ile Val Asp Gln Ala Arg Asn
   195 200 205
   Gly Ala Asp Phe Gly Lys Leu Ala Ile Ala His Ser Ala Asp Gln Gln
   210 215 220
   Ala Leu Asn Gly Gly Gln Met Gly Trp Gly Arg Ile Gln Glu Leu Pro
   225 230 235 240
   Gly Ile Phe Ala Gln Ala Leu Ser Thr Ala Lys Lys Gly Asp Ile Val
   245 250 255
   Gly Pro Ile Arg Ser Gly Val Gly Phe His Ile Leu Lys Val Asn Asp
   260 265 270
   Leu Arg Gly Glu Ser Lys Asn Ile Ser Val Thr Glu Val His Ala Arg
   275 280 285
   His Ile Leu Leu Lys Pro Ser Pro Ile Met Thr Asp Glu Gln Ala Arg
   290 295 300
   Val Lys Leu Glu Gln Ile Ala Ala Asp Ile Glu Ser Gly Lys Thr Thr
   305 310 315 320
   Phe Ala Ala Ala Thr Lys Glu Phe Ser Gln Asp Pro Val Ser Ala Asn
                   325                 330                 335
   Gln Gly Gly Asp Leu Gly Trp Ala Thr Pro Asp Ile Phe Asp Pro Ala
               340                 345                 350
   Phe Arg Asp Ala Leu Thr Arg Leu Asn Lys Gly Gln Met Ser Ala Pro
           355                 360                 365
   Val His Ser Ser Phe Gly Trp His Leu Ile Glu Leu Leu Asp Thr Arg
       370                 375                 380
   Asn Val Asp Lys Thr Asp Ala Ala Gln Lys Asp Arg Ala Tyr Arg Met
   385                 390                 395                     400
   Leu Met Asn Arg Lys Phe Ser Glu Glu Ala Ala Ser Trp Met Gln Glu
                   405                 410                 415
   Gln Arg Ala Ser Ala Tyr Val Lys Ile Leu Ser Asn
               420                 425